(19) **Europäisches Patentamt / European Patent Office / Office européen des brevets**

(11) **EP 1 889 824 B1**

(12) **EUROPEAN PATENT SPECIFICATION**

(45) Date of publication and mention
of the grant of the patent:
**23.11.2011 Bulletin 2011/47**

(51) Int Cl.:
*C07C 5/48* (2006.01)   *C07C 5/333* (2006.01)
*C07C 15/46* (2006.01)   *C07B 61/00* (2006.01)

(21) Application number: **06766556.2**

(22) Date of filing: **09.06.2006**

(86) International application number:
**PCT/JP2006/311644**

(87) International publication number:
**WO 2006/132370 (14.12.2006 Gazette 2006/50)**

(54) **PROCESS FOR PRODUCTION OF STYRENE**

VERFAHREN ZUR HERSTELLUNG VON STYROL

PROCÉDÉ DE PRODUCTION DU STYRÈNE

(84) Designated Contracting States:
**CZ**

(30) Priority: **10.06.2005   JP 2005171108**

(43) Date of publication of application:
**20.02.2008   Bulletin 2008/08**

(73) Proprietor: **Mitsubishi Chemical Corporation
Tokyo 108-0014 (JP)**

(72) Inventors:
  • **OBAYASHI, Shuji c/o Mitsubishi Chemical
    3140102 (JP)**
  • **SUZUKI, Shohei c/o Mitsubishi Chemical
    3140102 (JP)**

  • **NISHIYAMA, Takahito c/o Mitsubishi Chemical
    5108530 (JP)**
  • **KINOSHITA, Hisao c/o Mitsubishi Chemical
    5108530 (JP)**
  • **ITO, Mitsunobu c/o Mitsubishi Chemical
    5108530 (JP)**

(74) Representative: **Merkle, Gebhard
TER MEER STEINMEISTER & PARTNER GbR,
Patentanwälte
Mauerkircherstrasse 45
81679 München (DE)**

(56) References cited:
**JP-A- 11 080 045       JP-A- 60 130 531
JP-A- 60 241 925       JP-A- 62 067 034
JP-A- 2002 154 991**

**Description**

TECHNICAL FIELD

The present invention relates to a process for producing styrene, particularly to a process for producing styrene by oxidative dehydrogenation of ethylbenzene, which is capable of producing styrene in high yield while preventing a decline of the activity of a dehydrogenation catalyst over a long period of time.

BACKGROUND ART

A process for producing styrene by dehydrogenating ethylbenzene by means of e.g. a potassium-containing iron-type dehydrogenation catalyst, has been industrially widely used. However, it is usually difficult to obtain high yield for such a reason that the dehydrogenation reaction is strongly restricted by equilibrium, or since the dehydrogenation reaction is an endothermic reaction, the reaction temperature decreases as the reaction proceeds, by the reaction in a heat insulation apparatus. Therefore, it has been proposed to combine the dehydrogenation step with an oxidation step of selectively oxidizing hydrogen formed by the dehydrogenation reaction by means of an oxidation catalyst.

At that time, the outflow gas from the dehydrogenation step contains an alkaline substance such as a potassium compound derived from the dehydrogenation catalyst. Therefore, when such an outflow gas is fed to the oxidation step, the alkaline substance will be deposited on the oxidation catalyst, whereby the oxidation selectivity will be hindered, and the combustion amount of hydrocarbons such as styrene, ethylbenzene, etc. in the oxidation step will increase to produce carbon dioxide in a large amount. On the other hand, during a steady reaction by a hydrogenation catalyst, the reaction activity decreases even by the presence of a very small amount of carbon dioxide, and it is known that carbon dioxide thus formed tends to reduce the activity of the dehydrogenation catalyst in the later stage to impair the yield of styrene.

In order to prevent such an impairment of the oxidation selectivity of hydrogen in the oxidation step and to prevent formation of carbon dioxide by combustion of hydrocarbons in the oxidation step, a method has been proposed wherein an alkaline substance in the reaction mixture to be fed to the oxidation step, is preliminarily removed (e.g. Patent Document 1). However, in the method of Patent Document 1, the alkaline substance will not be supplied to the later stage dehydrogenation step on the downstream side of the oxidation step, whereby an impairment with time of the dehydrogenation catalyst performance in the later stage will be accelerated, the reaction activity will be impaired, and further the selectivity will also be impaired to form a large amount of carbon dioxide by e.g. a steam reforming reaction, thus leading to a vicious circle such that by the influence, the activity of the dehydrogenation catalysts will be further impaired.

Further, a method of maintaining such a production rate of carbon dioxide in the dehydrogenation step to be less than 2.1 times as compared with the initial stage of the reaction, has also been proposed (e.g. Patent Document 2). However, by a study by the present inventors, it has been found that by the method of Patent Document 2, it is not possible to prevent an increase of the production amount of carbon dioxide by combustion of hydrocarbons from a place where the oxygen-containing gas is mixed to the inlet of the oxidation step, and it is not possible to continue the styrene production in high yield.

Further, although not directed to prevent formation of carbon dioxide by combustion of hydrocarbons in the oxidation step, a method has been also proposed wherein an outflow gas from the dehydrogenation step is preliminarily cooled by direct or indirect heat exchange, and the heating amount in the oxidation step is increased as compared with a case where no cooling is carried out, thereby to increase the hydrogen combustion amount in the oxidation step (e.g. Patent Document 3). However, by a study by the present inventors, it has been found that when the flowout gas is cooled, the contained alkaline substance will be deposited on the inner wall of the flow channel, and on such deposited inner wall, the combustion amount of hydrocarbons such as styrene, ethylbenzene, etc. will increase to form a large amount of carbon dioxide, whereby it is not possible to continue the styrene production in high yield.

　　Patent Document 1: JP-A-11-80045
　　Patent Document 2: JP-A-2002-154991
　　Patent Document 3: JP-A-4-20410

DISCLOSURE OF THE INVENTION

OBJECT TO BE ACCOMPLISHED BY THE INVENTION

In view of the above described prior art, the present invention has been made to prevent formation of carbon dioxide by the combustion reaction of hydrocarbons due to deposition of an alkaline substance contained in the dehydrogenation reaction gas from the dehydrogenation step on the inner wall of the flow channel from a place where the dehydrogenation reaction gas is mixed with an oxygen-containing gas to the inlet of the subsequent oxidation step.

It is an object of the present invention to provide a process for producing styrene by dehydrogenation of ethylbenzene by means of a combination of a dehydrogenation reaction and an oxidation reaction, wherein the formation of carbon dioxide is prevented in the section from a place where the dehydrogenation reaction gas is mixed with the oxygen-containing gas to the inlet of the oxidation step, thereby to produce styrene in high yield for a long period.

MEANS TO ACCOMPLISH THE OBJECT

The present inventors have conducted an extensive study to solve the above problem and as a result, have found it possible to accomplish the above object by controlling the conversion of oxygen by combustion to be at most a specific level in the section from the place where the dehydrogenation reaction gas is mixed with the oxygen-containing gas to the inlet of the oxidation step, and thus have arrived at the present invention. Namely, the present invention provides a process for producing styrene, which comprises the following steps (1) to (3) and which is characterized in that at the time of feeding a dehydrogenation reaction gas obtained in step (1) and mixed with an oxygen-containing gas, to step (2), the conversion of oxygen by combustion is controlled to be at most 15% in the section from the place where the dehydrogenation reaction gas is mixed with the oxygen-containing gas to the inlet of step (2):

Step (1): a dehydrogenation step of subjecting a raw material gas comprising at least ethylbenzene and steam to a dehydrogenation reaction of the ethylbenzene in the presence of a dehydrogenation catalyst, to obtain a dehydrogenation reaction gas comprising styrene, hydrogen, unreacted ethylbenzene, steam and an alkaline substance derived from the dehydrogenation catalyst,

Step (2): an oxidation step of subjecting the dehydrogenation reaction gas obtained in the dehydrogenation step to an oxidation reaction of at least a part of hydrogen in the presence of an oxidation catalyst and in the coexistence of an oxygen-containing gas to obtain an oxidation reaction gas, and

Step (3): a dehydrogenation step of subjecting the oxidation reaction gas obtained in the oxidation step to a dehydrogenation reaction of ethylbenzene in the presence of a dehydrogenation catalyst to obtain a dehydrogenation reaction gas comprising styrene, hydrogen, unreacted ethylbenzene, steam, and an alkaline substance derived from the dehydrogenation catalyst.

EFFECTS OF THE INVENTION

According to the present invention, it is possible to provide a process for producing styrene by dehydrogenation of ethylbenzene by means of a combination of a dehydrogenation reaction and an oxidation reaction, wherein the formation of carbon dioxide is prevented in the section from the place where the dehydrogenation reaction gas is mixed with the oxygen-containing gas to the inlet of the oxidation step, thereby to produce styrene in high yield for a long period.

BEST MODE FOR CARRYING OUT THE INVENTION

In the process for producing styrene of the present invention, step (1) is a dehydrogenation step of subjecting a raw material gas comprising at least ethylbenzene and steam to a dehydrogenation reaction of the ethylbenzene in the presence of a dehydrogenation catalyst, to obtain a dehydrogenation reaction gas comprising styrene, hydrogen, unreacted ethylbenzene, steam and an alkaline substance derived from the dehydrogenation catalyst.

In the dehydrogenation step (1), ethylbenzene as a raw material hydrocarbon is fed to this dehydrogenation step (1) in the form of a gas mixed with steam. The raw material hydrocarbon may contain, in addition to ethylbenzene, other hydrocarbons such as styrene, toluene, benzene, etc. The ethylbenzene concentration is usually at least 90%, preferably at least 95%, more preferably at least 97%. Further, the blend ratio of steam to the raw material hydrocarbon including ethylbenzene is usually within a range of from 1 to 15, preferably within a range of from 1 to 10, by molar ratio.

The dehydrogenation catalyst is not particularly limited. However, usually, one disclosed in e.g. JP-A-60-130531, i.e. an iron type catalyst containing an alkali metal or alkaline earth metal, or one having other metal such as zirconium, tungsten, molybdenum, vanadium or chromium, incorporated to such an iron type catalyst, may be employed. Among them, a potassium-containing iron type catalyst containing iron oxide as the main component and having potassium oxide and optionally the above mentioned other metal, etc. incorporated thereto, is preferred. As an example, one disclosed in JP-A-4-277030, i.e. one containing iron oxide and potassium oxide as main components and having titanium oxide incorporated as a cocatalyst component, may, for example, be mentioned.

The reaction temperature in the dehydrogenation step (1) is usually at least 500°C, preferably at least 550°C and usually at most 700°C, preferably at most 670°C. The dehydrogenation reaction of ethylbenzene is an endothermic reaction, whereby as the reaction proceeds, the temperature in the step (1) decreases. The pressure is usually within a range of from 0.0049 to 0.98 MPa.

3

In the dehydrogenation step (1), ethylbenzene is dehydrogenated to form styrene and hydrogen, whereby a dehydrogenation reaction gas comprising styrene, hydrogen, unreacted ethylbenzene, steam and an alkaline substance derived from the dehydrogenation catalyst, is obtained.

In the present invention, "an alkaline substance" is a generic term for a compound containing an alkaline metal, such as an oxide, carbonate or hydroxide of an alkali metal or alkaline earth metal. The alkaline substance contained in the dehydrogenation reaction gas is not identified, but is assumed to be a hydroxide of an alkaline metal such as potassium hydroxide or a carbonate of an alkaline metal such as potassium carbonate, since it is formed in the presence of high temperature steam and carbon dioxide.

The apparatus to be used for the dehydrogenation reaction in the dehydrogenation step (1) in the present invention is not particularly limited, but, usually, a fixed bed apparatus having a dehydrogenation catalyst-packed layer is employed.

The dehydrogenation reaction gas discharged from this dehydrogenation step (1) has a low temperature as compared with the inlet of the dehydrogenation step, and such a dehydrogenation reaction gas is fed, after being mixed with the oxygen-containing gas, to the oxidation step (2).

Here, the oxygen-containing gas is not particularly limited so long as it is a gas containing oxygen. It may, for example, be air, diluted air, oxygen-enriched air, oxygen diluted with an inert gas, or the like. The method for feeding the oxygen-containing gas is not particularly limited, and the oxygen-containing gas may be fed to the dehydrogenation reaction gas discharged from the dehydrogenation step (1), and such a mixed gas is fed to the oxidation step (2).

In the process for producing styrene of the present invention, step (2) is an oxidation step of subjecting the dehydrogenation reaction gas obtained in the dehydrogenation step (1) to an oxidation reaction of at least a part of hydrogen in the presence of an oxidation catalyst and in the coexistence of an oxygen-containing gas.

In the oxidation step (2), as the oxidation catalyst, an optional one may be employed so long as it is one capable of selectively burning hydrogen in the coexistence of styrene and ethylbenzene. Usually, a noble metal type oxidation catalyst is employed. For example, it is a catalyst disclosed in e.g. JP-A-60-130531, i.e. a catalyst comprising platinum and potassium, or a catalyst comprising platinum, tin and potassium. Further, a catalyst disclosed in e.g. JP-A-61-225140, i.e. a catalyst comprising an alkali metal or alkaline earth metal, Group 4A such as germanium, tin or lead, and a noble metal, may, for example, be mentioned. Further, a catalyst disclosed in JP-A-11-322303, i.e. a catalyst comprising platinum and niobium or tantalum, may, for example, be also employed.

The apparatus to be used for the oxidation reaction in the oxidation step (2) in the present invention is not particularly limited. Usually, a fixed bed reactor having an oxidation catalyst-packed layer is employed, and the oxidation reaction gas discharged from the oxidation step (2) has a temperature increased by the heat of the oxidation reaction of hydrogen as compared with the dehydrogenation reaction gas. It is usually within a range of from 500 to 700°C, preferably from 550 to 670°C. The reaction gas discharged from the oxidation step (2) will then be fed to the dehydrogenation step (3). In the oxidation step (2), only the temperature rises due to the heat of the oxidation reaction of hydrogen, but also hydrogen decreases as it is oxidized, whereby there will be a merit such that in the subsequent dehydrogenation step (3), the equilibrium hindrance of the dehydrogenation reaction tends to be less, and the dehydrogenation reaction will be accelerated.

In the process for producing styrene of the present invention, step (3) is a dehydrogenation step of subjecting the oxidation reaction gas obtained in the oxidation step (2) to a dehydrogenation reaction of ethylbenzene in the presence of a dehydrogenation catalyst to obtain a dehydrogenation reaction gas comprising styrene, hydrogen, unreacted ethylbenzene, steam and an alkaline substance derived from the dehydrogenation catalyst.

The catalyst, the reaction conditions, the apparatus, etc. to be used for the dehydrogenation reaction in the dehydrogenation step (3) may optionally be selected under the conditions described with respect to the dehydrogenation step (1), and step (3) is carried out independently from the dehydrogenation step (1).

The process for producing styrene of the present invention comprises the above mentioned steps (1) to (3) and is essentially characterized in that at the time of feeding a dehydrogenation reaction gas obtained in step (1) and mixed with an oxygen-containing gas, to step (2), the conversion of oxygen by combustion is controlled to be at most 15% in the section from the place where the dehydrogenation reaction gas is mixed with the oxygen-containing gas to the inlet of step (2).

Further, in the present invention, if necessary, after the dehydrogenation step as step (3), an oxidation step as step (2') and a dehydrogenation step as step (3') may further be combined in a multistage fashion. Also in such a case, before feeding the dehydrogenation reaction gas obtained in the dehydrogenation step as step (3) to the oxidation step as step (2'), an oxygen-containing gas is mixed, and at that time, it is preferred to control the conversion of oxygen by combustion to be at most 15% in the section from the place where the oxygen-containing gas is mixed to the dehydrogenation reaction gas from the dehydrogenation step as step (3) to the inlet of step (2').

Usually, in a process for producing styrene by dehydrogenation of ethylbenzene by means of a combination of a dehydrogenation reaction and an oxidation reaction, an alkaline substance derived from a dehydrogenation catalyst in the dehydrogenation step (1), flies in the form of a vapor by the vapor pressure from step (1), and when the vapor pressure decreases due to e.g. a decrease of the dehydrogenation reaction gas temperature, the alkaline substance will be deposited on the inner wall of the flow channel such as the piping. If the alkaline substance is deposited on the inner wall of the flow channel, from the outlet of the dehydrogenation step (1) to before the place where the oxygen-containing gas is mixed, no oxygen is present and no combustion of hydrocarbons such as styrene, ethylbenzene, etc. takes place. On the other hand, in the section from the place where the oxygen-containing gas is mixed to the dehydrogenation reaction gas from the dehydrogenation step (1) to the inlet of the oxidation step (2), an alkaline substance is deposited on the inner wall of the flow channel, whereby the combustion amount of hydrocarbons such as styrene, ethylbenzene, etc. will increase to form a large amount of carbon dioxide, and such carbon dioxide impairs the activity of the dehydrogenation catalyst in the subsequent stage dehydrogenation step (3) thereby to lower the yield of styrene.

Whereas, in the present invention, in the section from the place where the dehydrogenation reaction gas is mixed with the oxygen-containing gas to the inlet of step (2), the conversion of oxygen by combustion is controlled to be at most 15%, preferably at most 10%, and the lower limit value is a conversion of at least 1% which takes place unavoidably, whereby styrene can be produced in high yield constantly for a long period.

In the present invention, the conversion of oxygen by combustion in the section from the place where the dehydrogenation reaction gas obtained in the dehydrogenation step (1) is mixed with the oxygen-containing gas to the inlet of the oxidation step (2), is one obtained by sampling the respective gases at the outlet of the dehydrogenation step (1) and at the inlet of the oxidation step (2) and analyzing such samples by gas chromatography, followed by calculation by the following formula:

$$\text{Conversion of oxygen by combustion} = [(A-B)/A] \times 100\ (\%)$$

A: The amount (mol) of oxygen mixed to the dehydrogenation reaction gas obtained in the dehydrogenation step (1)
B: The amount (mol) of oxygen at the inlet of the oxidation step (2)

In the present invention, the method for controlling the conversion of oxygen by combustion to be within the above mentioned range, is not particularly limited, so long as the conversion of oxygen by combustion can be consequently made to be within the above range.

For example, (i) a method of controlling the deposition on the inner wall of the flow channel, of an alkaline substance flying from the dehydrogenation step (1), (ii) a method of controlling formation of combustion reaction active sites even when an alkaline substance flying from the dehydrogenation step (1) is deposited on the inner wall of the flow channel, or (iii) a method which is capable of controlling the oxidation reaction of hydrocarbons even when an alkaline substance flying from the dehydrogenation step (1) is deposited on the inner wall of the flow channel and combustion reaction active sites are formed, may, for example, be mentioned.

The above method (i) may specifically be to control so that no decrease in temperature takes place between the outlet of the dehydrogenation step (1) and the inlet of the subsequent oxidation step (2), so that no decrease in vapor pressure of the alkaline substance takes place. For this purpose, a method is preferred wherein between the outlet of the dehydrogenation step (1) and the inlet of the oxidation step (2), particularly from the place where the oxygen-containing gas is mixed to the inlet of the oxidation step (2), the temperature of the dehydrogenation reaction gas is maintained at a level of at least the same temperature as the temperature of the outlet of the dehydrogenation step (1). Even by such an operation, in a case where an alkaline substance is already deposited on the inner wall of the flow channel, the desired effect can not be attained, and therefore, it is desired, for example, to take a step of preliminarily removing the alkaline substance from the inner wall of the flow channel at the time of e.g. an exchange of the catalyst.

However, in a case where the temperature from the outlet of the dehydrogenation step (1) to the inlet of the oxidation step (2) is made higher than the temperature at the outlet of the dehydrogenation step (1), the temperature-rising width in the oxidation step (2) tends to be small (the amount of feed of oxygen required in the oxidation step tends to be small), whereby the hydrogen combustion rate tends to decrease. Consequently, in the subsequent stage dehydrogenation step (3), the equilibrium hindrance against the dehydrogenation reaction of ethylbenzene tends to increase, whereby the conversion in the dehydrogenation reaction of ethylbenzene tends to decrease. If in order to avoid such a trouble, the amount of oxygen to be mixed is not decreased, the temperature at the outlet of the oxidation step (2) i.e. at the inlet of the subsequent dehydrogenation step (3) will be high, whereby the conversion in the dehydrogenation reaction of ethylbenzene in the dehydrogenation step (3) may become temporarily high, but flying of an alkaline substance derived from the dehydrogenation catalyst will be promoted. Namely, the deactivation rate is promoted, whereby it becomes difficult to produce styrene in high yield for a long period.

Accordingly, in a case where the temperature of the dehydrogenation reaction gas is controlled to be at a level of at least the same temperature as the temperature at the outlet of the dehydrogenation step (1), between the outlet of the dehydrogenation step (1) and the inlet of the oxidation step (2), particularly from the place where the oxygen-containing gas is mixed to the inlet of the oxidation step (2), the width of the temperature to be raised is adjusted to be usually at most 20°C, preferably at most 10°C, more preferably at most 5°C.

The following method may be mentioned in order to maintain the temperature of the dehydrogenation reaction gas to be at a level of at least the same temperature as the temperature at the outlet of the dehydrogenation step (1), between the outlet of the dehydrogenation step (1) and the inlet of the oxidation step (2), particularly from the place where the oxygen-containing gas is mixed to the inlet of the oxidation step (2). Namely, to the dehydrogenation reaction gas, a fluid having a temperature higher than the dehydrogenation reaction gas is mixed before mixing the oxygen-containing gas, or a heat exchanger is set before the place where the oxygen-containing gas is mixed, to carry out heat exchange with a high temperature fluid or, to the oxygen-containing gas, an inert gas such as steam is preliminarily mixed, and the temperature of such an inert gas is adjusted depending upon the change in the outlet temperature of the dehydrogenation step (1).

As an example of the above (ii), the following method may be mentioned.

Firstly, when the inner wall of the flow channel is made of a metal, the increase of the combustion amount of hydrocarbons by the deposition of an alkaline substance on the inner wall of the flow channel, is caused by an increase of the metal surface area of the inner wall of the flow channel by corrosion of the metal of the inner wall due to deposition on the inner wall of the flow channel, of the alkaline substance flying from the dehydrogenation step (1). Namely, it is assumed to take place by an increase of the contact amount of the process fluid with a substance to form active sites for the combustion reaction, e.g. a substance such as nickel.

Therefore, in order to suppress the formation of combustion reaction active sites, a method may be mentioned wherein as the material constituting the inner wall of the flow channel, a material hardly susceptible to corrosion by an alkaline substance is used, or a layer of a material hardly susceptible to corrosion is formed by coating, plating or flame spraying such a material on the surface of the inner wall of the flow channel. The material hardly susceptible to corrosion by an alkaline substance may, for example, be an inorganic material such as ceramics, an organic material such as a heat resistant resin, or a metal having a small content of nickel or the like. It is preferred to employ a metal having a small content of nickel or the like, from the viewpoint of availability and simplicity of its production. The nickel content in the metal to be used here is preferably less than 8%, more preferably at most 5%, further preferably at most 3%, particularly preferably at most 1%.

In a case where such a metal is used as a material for the inner wall of the flow channel, it is effective to treat the surface of the inner wall of the flow channel by buffing or electrolytic polishing to make the metal surface of the inner wall of the flow channel smooth to reduce the surface area thereby to prevent deposition of the alkaline substance and suppress corrosion. Here, "the inner wall of a flow channel" includes not only the inner wall surface of piping, but also all portions in contact with the fluid, such as the surface of a mixer to mix the dehydrogenation reaction gas and the oxygen-containing gas, the surface of a screen at the inlet of the catalyst layer of the oxidation step (2), etc.

Further, the following method may be mentioned as an example of the above (iii). Namely, the combustion reaction of hydrocarbons at the inner wall of the flow channel having the alkaline substance deposited thereon, is usually promoted when the temperature is higher, just like a usual oxidation reaction. Accordingly, specifically, it is a method of lowering the temperature of the dehydrogenation reaction gas between the outlet of the dehydrogenation step (1) and the inlet of the oxidation step (2), particularly from the place where the oxygen-containing gas is mixed to the dehydrogenation reaction gas to the inlet of the oxidation step (2).

However, in a case where the temperature from the outlet of the dehydrogenation step (1) to the inlet of the oxidation step (2) is substantially lowered than the temperature at the outlet of the dehydrogenation step (1), the temperature-rising width in the oxidation step (2) will be large, and the amount of oxygen to be mixed is required to be increased. Namely, the combustion reaction amount of hydrocarbons in the oxidation step (2) will increase, and the amount of formation of carbon dioxide will also increase. As a result, in the subsequent dehydrogenation step (3), the conversion in the dehydrogenation reaction of ethylbenzene will decrease. If the amount of oxygen to be mixed is not increased in order to avoid such a drawback, the temperature at the outlet of the oxidation step (2), i.e. at the inlet of the subsequent dehydrogenation step (3) tends to be low. Consequently, the conversion in the dehydrogenation reaction of ethylbenzene in the dehydrogenation step (3) tends to be low, and it becomes difficult to produce styrene in high yield for a long period. Accordingly, the temperature of the dehydrogenation reaction gas between the outlet of the dehydrogenation step (1) and the inlet of the oxidation step (2), particularly from the place where the oxygen-containing gas is mixed to the dehydrogenation reaction gas to the inlet of the oxidation step (2), is preferably at most 560°C, more preferably at most 550°C, but it is preferably at least 500°C. Further, the temperature-rising width in the oxidation step (2) is preferably maintained to be from 10 to 100°C, more preferably from 25 to 95°C.

There is a method to maintain the temperature of the dehydrogenation reaction gas to be within the above range, between the outlet of the dehydrogenation step (1) and the inlet of the oxidation step (2), particularly from the place where the oxygen-containing gas is mixed to the inlet of the oxidation step (2). Namely, a method may be mentioned wherein to the dehydrogenation reaction gas, a fluid having a temperature lower than the dehydrogenation reaction gas is mixed prior to mixing the oxygen-containing gas, or a heat exchanger is installed before the place where the oxygen-containing gas is mixed, to carry out heat exchange with a fluid having a low temperature, or an inert gas such as steam is preliminarily mixed to the oxygen-containing gas, and the temperature of the inert gas is adjusted depending upon the change of the outlet temperature of the dehydrogenation step (1).

Further, in the present invention, another method to maintain the conversion of oxygen by combustion to be within the above range may, for example, be a method wherein from the place where the oxygen-containing gas is mixed to the dehydrogenation reaction gas to the inlet of the oxidation step (2), the surface area of the flow channel is reduced to minimize the contact time of the dehydrogenation reaction gas with the combustion reaction active sites.

EXAMPLES

Now, the present invention will be described in further detail with reference to Examples, but it should be understood that the present invention is by no means restricted by the following Examples.

EXAMPLE 1

An apparatus was employed wherein five stages of fixed bed flow reactors, each reactor has a reaction tube having an inner diameter of 81.1 mm equipped with a thermocouple insertion tube having an outer diameter of 12 mm, were connected in series by piping. In the first stage, third stage and fifth stage reactors, an iron oxide type catalyst ("Styromax Plus-5", manufactured by Süd-Chemie Catalysts Japan, Inc.) was packed as a commercially available dehydrogenation catalyst for the production of styrene, and in the second stage and fourth stage reactors, an oxidation catalyst prepared in accordance with Example 8 in JP-A-9-29095, was packed. The packed amounts of the dehydrogenation catalyst were 4.8 L in the first stage, 4.8 L in the third stage and 9.6 L in the fifth stage, and the packed amounts of the oxidation catalyst were 1.3 L in the second stage, and 1.3 L in the fourth stage.

Further, between the first stage reactor and the second stage reactor, a feeding pipe was installed to mix a gas comprising air and steam to the dehydrogenation reaction gas from the first stage dehydrogenation reactor. Likewise, between the third stage reactor and the fourth stage reactor, a feeding pipe was installed to mix a gas comprising air and steam to the dehydrogenation reaction gas from the third stage reactor.

The respective reactors were installed in separate electric furnaces and heated while nitrogen gas was fed at a rate of 100 NL/min from the inlet of the first stage reactor and at a rate of 10 NL/min from each of the feeding pipes for a gas comprising air and steam, installed between the first stage and second stage reactors and between the third stage and fourth stage reactors. When the temperatures at the outlets of the first, third and fifth stage dehydrogenation catalyst layers all reached at least 300°C, the nitrogen gas was switched to steam. The steam was fed at a rate of 5.7 kg/hr from the inlet of the first stage reactor and at a rate of 0.8 kg/hr from each of the feeding pipes to mix a gas comprising air and steam, installed between the first stage and second stage reactors and between the third stage and fourth stage reactors.

When the temperature at the inlet of the first stage dehydrogenation catalyst layer reached 580°C, the gas fed from the inlet of the first stage reactor was switched to a mixed gas comprising steam and ethylbenzene. The composition of the feed gas was steam:ethylbenzene = 10:1 (molar ratio). Then, air was mixed to steam which was mixed to the dehydrogenation reaction gas of the first stage reactor. Likewise, air was mixed to steam which was mixed to the dehydrogenation reaction gas of the third stage reactor. The flow rate of ethylbenzene fed to the first stage reactor was set so that the space velocities (LHSV: Liquid Hourly Space Velocity) of the respective catalyst layers would be 1.2 hr$^{-1}$ in the first stage, 4.4 hr$^{-1}$ in the second stage, 1.2 hr$^{-1}$ in the third stage, 4.4 hr$^{-1}$ in the fourth stage and 0.6 hr$^{-1}$ in the fifth stage. The reaction was carried out by adjusting the temperature of steam to be mixed to ethylbenzene so that the inlet temperature of the first stage dehydrogenation reactor would be 580°C and adjusting the flow rate of air to be mixed to the dehydrogenation reaction gas of the first stage and third stage reactors, so that the inlet temperatures of the third stage and fifth stage dehydrogenation reactors would be 580°C. Further, the reaction pressure was set to be 0.045 MPa at the outlet of the fifth stage reactor.

Upon expiration of 70 hours by feeding ethylbenzene, the composition of the gas fed from the inlet of the first stage reactor was changed to steam:ethylbenzene = 7:1 (molar ratio). The temperature of steam to be mixed to ethylbenzene was adjusted so that the inlet temperature of the first stage dehydrogenation reactor would be 592°C. The flow rate of air to be mixed to the dehydrogenation reaction gas from the first stage reactor was adjusted so that the inlet temperature of the third stage dehydrogenation reactor would be 613°C. Further, the temperature of steam to be mixed was adjusted so that the gas temperature after mixing the mixed gas comprising air and steam would be higher by 5°C than the temperature of the dehydrogenation reaction gas at the outlet of the first stage dehydrogenation reactor. Likewise, the flow rate of air to be mixed to the dehydrogenation reaction gas from the third stage reactor was adjusted so that the inlet temperature of the fifth stage dehydrogenation reactor would be 631°C. Further, the temperature of steam to be mixed was adjusted so that the gas temperature after mixing the mixed gas comprising air and steam would be higher by 5°C than the temperature of the dehydrogenation reaction gas at the outlet of the third stage dehydrogenation reactor.

Here, the inlet temperature of the dehydrogenation reactor was measured at 20 mm upstream of the inlet of the catalyst layer, and the temperature after mixing the mixed gas comprising air and steam was measured at 20 mm upstream of the inlet of the oxidation catalyst layer of the oxidation reactor.

Every time upon expiration of the time indicated in Table 1 from the initiation of the reaction, the gas at the outlet of each reactor and ethylbenzene to be fed to the first stage dehydrogenation reactor were sampled, and the compositions were analyzed by gas chromatography. The type of the gas chromatography and the column used, were as follows.

Type: GC-14B

Column:

(1) MS-5A for hydrogen analysis
(2) Chromosorb-W for analyses of benzene, toluene, ethylbenzene and styrene
(3) Porapak-Q for analyses of carbon dioxide, ethane, ethylene and water
(4) MS-13X for analyses of oxygen, nitrogen, methane and carbon monoxide

The results of the reaction for 13,000 hours after initiation of the feed of ethylbenzene to the inlet of the first stage reactor, are shown in Table 1.

COMPARATIVE EXAMPLE 1

The temperature of steam to be mixed was adjusted so that the gas temperature after mixing the mixed gas comprising air and steam would be lower by 5°C than the temperature of the dehydrogenation reaction gas from the first stage dehydrogenation reactor.

Likewise, the temperature of steam to be mixed was adjusted so that the gas temperature after mixing the mixed gas comprising air and steam would be lower by 10°C than the temperature of the dehydrogenation reaction gas from the third stage dehydrogenation reactor (in reality, the temperature became lower by from 8 to 11°C). Otherwise, the reaction was carried out in the same manner as in Example 1. The results of the reaction for 13,000 hours after initiation of the feed of ethylbenzene to the inlet of the first stage reactor, are shown in Table 1.

EXAMPLE 2

The temperature of steam to be mixed was adjusted so that the gas temperature after mixing the mixed gas comprising air and steam would be lower by 5°C than the temperature of the dehydrogenation reaction gas from the first stage dehydrogenation reactor. Further, after the time point (upon expiration of 10,000 hours from the initiation of the reaction) when the gas temperature after mixing the mixed gas comprising air and steam exceeded 560°C, the temperature of steam to be mixed was adjusted so that the temperature at such a portion would be 545°C.

Likewise, the temperature of steam to be mixed was adjusted so that the gas temperature after mixing the mixed gas of air and steam would be lower by 10°C than the temperature of the dehydrogenation reaction gas from the third stage dehydrogenation reactor (in reality, the temperature became lower by 8°C). After the time point (upon expiration of 3,500 hours from the initiation of the reaction) when the gas temperature after mixing the mixed gas comprising air and steam exceeded 560°C, the temperature of steam to be mixed was adjusted so that the temperature at such a portion would be 545°C. Otherwise, the reaction was carried out in the same manner as in Example 1. The results of the reaction for 13,000 hours after initiation of the feed of ethylbenzene to the inlet of the first stage reactor, are shown in Table 1.

TABLE 1

| Reaction time (hrs) | | | 3,000 | 5,000 | 8,000 | 11,000 | 13,000 |
|---|---|---|---|---|---|---|---|
| Ex. 1 | Inlet temperature of dehydrogenation reactor (°C) | First stage reactor | 592 | 600 | 605 | 628 | 628 |
| | | Third stage reactor | 613 | 619 | 623 | 638 | 639 |
| | | Fifth stage reactor | 631 | 636 | 640 | 640 | 640 |
| | Outlet temperature of dehydrogenation reactor (°C) | First stage reactor | 541 | 544 | 549 | 567 | 570 |
| | | Third stage reactor | 569 | 579 | 587 | 605 | 607 |
| | Inlet temperature of oxidation reactor (°C) | Second stage reactor | 546 | 549 | 554 | 572 | 575 |
| | | Fourth stage reactor | 574 | 584 | 592 | 610 | 612 |
| | Conversion of oxygen by combustion at air-mixing portion (%) | Second stage reactor | 3.2 | 4.3 | 4.7 | 5.2 | 5.6 |
| | | Fourth stage reactor | 5.8 | 6.5 | 7.3 | 8.3 | 8.7 |
| | Styrene yield (wt%) | | 68.5 | 67.7 | 64.9 | 64.8 | 63.5 |
| Comp. Ex. 1 | Inlet temperature of dehydrogenation reactor (°C) | First stage reactor | 592 | 600 | 605 | 628 | 628 |
| | | Third stage reactor | 613 | 619 | 623 | 638 | 639 |
| | | Fifth stage reactor | 631 | 636 | 640 | 640 | 640 |
| | Outlet temperature of dehydrogenation reactor (°C) | First stage reactor | 541 | 544 | 549 | 567 | 570 |
| | | Third stage reactor | 568 | 578 | 586 | 607 | 610 |
| | Inlet temperature of oxidation reactor (°C) | Second stage reactor | 536 | 539 | 544 | 562 | 565 |
| | | Fourth stage reactor | 559 | 570 | 578 | 597 | 599 |
| | Conversion of oxygen by combustion at air-mixing portion (%) | Second stage reactor | 2.6 | 3.2 | 4.1 | 15.1 | 18.5 |
| | | Fourth stage reactor | 6.0 | 17.5 | 26.1 | 40.2 | 45.2 |
| | Styrene yield (wt%) | | 68.4 | 66.8 | 63.5 | 62.0 | 60.3 |
| Ex. 2 | Inlet temperature of dehydrogenation reactor (°C) | First stage reactor | 592 | 600 | 605 | 628 | 628 |
| | | Third stage reactor | 613 | 619 | 623 | 638 | 639 |
| | | Fifth stage reactor | 631 | 636 | 640 | 640 | 640 |
| | Outlet temperature of dehydrogenation reactor (°C) | First stage reactor | 541 | 544 | 549 | 567 | 570 |
| | | Third stage reactor | 568 | 578 | 586 | 604 | 606 |
| | Inlet temperature of oxidation reactor (°C) | Second stage reactor | 536 | 539 | 544 | 545 | 545 |
| | | Fourth stage reactor | 559 | 545 | 545 | 545 | 545 |
| | Conversion of oxygen by combustion at air-mixing portion (%) | Second stage reactor | 2.7 | 3.2 | 4.2 | 4.6 | 4.9 |
| | | Fourth stage reactor | 5.9 | 4.3 | 4.8 | 4.8 | 5.0 |
| | Styrene yield (wt%) | | 68.3 | 68.0 | 65.5 | 65.3 | 64.1 |

The respective items in Table 1 show the following values.

"Reaction time": The time passed from initiation of the feed of ethylbenzene to the first stage reactor.

"Inlet temperature of oxidation reactor (second stage reactor)": The temperature from the place where the mixed gas comprising air and steam was mixed to the reaction gas from the outlet of the first stage dehydrogenation reactor to the inlet of the second stage oxidation reactor.

"Inlet temperature of oxidation reactor(fourth stage reactor)": The temperature from the place where the mixed gas comprising air and steam was mixed to the reaction gas from the outlet of the third stage dehydrogenation reactor to the inlet of the fourth stage oxidation reactor.

"Conversion of oxygen by combustion at air-mixing portion (second stage reactor)": The conversion of oxygen by combustion at the metal piping surface and at the space from the place where the mixed gas comprising air and steam was mixed to the reaction gas from the outlet of the first dehydrogenation reactor to the inlet of the second stage oxidation reactor. It was calculated by the following formula. Conversion of oxygen by combustion at air-mixing portion (second stage reactor) = [(A1-B2)/A1] $\times$ 100 (%)

A1: Amount (mols) of oxygen to be mixed to the dehydrogenation reaction gas from the first stage dehydrogenation reactor.
B2: Amount (mols) of oxygen at the inlet of the second stage oxidation reactor.

"Conversion of oxygen by combustion at air-mixing portion (fourth stage reactor)": The conversion of oxygen by combustion at the metal surface and the space from a place where the mixed gas comprising air and steam was mixed to the reaction gas from the outlet of the third stage dehydrogenation reactor to the inlet of the fourth oxidation reactor. It was calculated by the following formula.

$$\texttt{Conversion of oxygen by combustion at air-mixing}$$
$$\texttt{portion (fourth stage reactor) = [(A3-B4)/A3]} \times \texttt{100 (\%)}$$

A3: Amount (mols) of oxygen to be mixed to the dehydrogenation reaction gas from the third stage dehydrogenation reactor.
B4: Amount (mols) of oxygen at the inlet of the fourth stage oxidation reactor.

"Styrene yield": The styrene yield in total from the first stage dehydrogenation reactor to the fifth stage dehydrogenation reactor, and it was calculated by the following formula.

$$\texttt{Styrene yield = [(Z-Y)/X]} \times \texttt{100 (wt\%)}$$

X: Amount (kg) of ethylbenzene fed to the first stage dehydrogenation reactor
Y: Amount (kg) of styrene fed to the first stage dehydrogenation reactor
Z: Amount (kg) of styrene discharged from the fifth stage dehydrogenation reactor

EXAMPLE 3

A wire of SUS410S (prescribed value of nickel content: at most 0.6%) having an outer diameter of 2 mm and a length of 10 mm was placed on a petri dish, and five droplets of a 5 wt% potassium hydroxide aqueous solution were applied by a dropper, whereupon the wire was put in a dryer and dried at 120°C for one hour. Then, the wire was taken out from the dryer, put into an electric furnace and baked at 640°C for 24 hours, whereupon it was taken out from the electric furnace and cooled to room temperature.
67.1 g of quartz chips having a particle size of from 2.4 to 6 mm were packed at the lower side of a quartz reaction tube having an inner diameter of 16 mm and a length of 500 mm, and the baked wire was packed thereon, and further, 50.6 g of quartz chips having a particle size of from 1 to 2.4 mm were packed thereon. The reaction tube was set in an electric furnace and heated while a mixed gas comprising nitrogen and hydrogen was fed at a rate of 0.09 NL/min. The composition of the mixed gas was nitrogen:hydrogen = 2.0:1.0 (molar ratio). When the temperature at the wall surface of the reactor reached 520°C, the temperature of the wall surface was maintained to be 520°C, and the reaction tube was maintained under such conditions for 30 minutes. Then, the feed gas was switched to a mixed gas comprising ethylbenzene, styrene, steam, hydrogen, oxygen and nitrogen, which was fed at a rate of 1.0 NL/min. The composition of the feed gas was ethylbenzene:styrene:steam:hydrogen:oxygen:nitrogen = 1.0:0.87:17.6:0.65:0.17:1.3 (molar ratio). Upon expiration of 30 minutes, the outlet gas of the reaction tube was sampled, and its composition was analyzed by gas chromatography.
Thereafter, only the temperature of the wall surface of the reactor was changed to 550°C, and upon expiration of 30 minutes, the outlet gas of the reaction tube was sampled, and its composition was analyzed by gas chromatography. Then, the temperature of the wall surface of the reaction tube was changed to 580°C and 610°C, and upon expiration of 30 minutes at each wall surface temperature, the outlet gas of the reaction tube was sampled and its composition was analyzed in the same manner. The results are shown in Table 2.
Further, by the following formula, the oxygen selectivity of other than hydrogen combustion at each temperature was calculated. The results are shown in Table 2. Here, the oxygen selectivity of other than hydrogen combustion represents the proportion of oxygen for combustion of ethylbenzene and styrene in the feed gas, among the oxygen used for combustion.

```
Oxygen selectivity of other than hydrogen combustion

= [1-(C-D)×0.5/(A-B)] × 100 (%)
```

A: Amount (mols) of oxygen fed to the reaction tube
B: Amount (mols) of oxygen at the outlet of the reaction tube
C: Amount (mols) of hydrogen fed to the reaction tube
D: Amount (mols) of hydrogen at the outlet of the reaction tube

COMPARATIVE EXAMPLE 2

A reaction was carried out in the same manner as in Example 3 except that a wire of SUS304 (prescribed value of nickel content: 8.0 to 10.5%) was used instead of SUS410S, and the oxygen selectivity of other than hydrogen combustion was calculated. The results are shown in Table 2.

TABLE 2

| Reaction temperature (°C) | | 520 | 550 | 580 | 610 |
|---|---|---|---|---|---|
| Ex. 3 | Oxygen selectivity of other than hydrogen combustion (%) | 0.50 | 0.64 | 0.83 | 1.3 |
| Comp. Ex. 2 | Oxygen selectivity of other than hydrogen combustion (%) | 2.4 | 4.0 | 6.1 | 9.5 |

INDUSTRIAL APPLICABILITY

The present invention provides a process for producing styrene by dehydrogenation of ethylbenzene, whereby the amount of formation of carbon dioxide is suppressed in the section from the place where the dehydrogenation reaction gas is mixed with an oxygen-containing gas to the inlet of an oxidation step, and styrene is produced in high yield for a long period.

**Claims**

1. A process for producing styrene, which comprises the following steps (1) to (3) and which is **characterized in that** at the time of feeding a dehydrogenation reaction gas obtained in step (1) and mixed with an oxygen-containing gas, to step (2), the conversion of oxygen by combustion is controlled to be at most 15% in the section from the place where the dehydrogenation reaction gas from the outlet of the first dehydrogenation reactor is mixed with the oxygen-containing gas to the inlet of the step (2) oxidation reactor:

   Step (1): a dehydrogenation step of subjecting a raw material gas comprising at least ethylbenzene and steam to a dehydrogenation reaction of the ethylbenzene in the presence of a dehydrogenation catalyst, to obtain a dehydrogenation reaction gas comprising styrene, hydrogen, unreacted ethylbenzene, steam and an alkaline substance derived from the dehydrogenation catalyst,
   Step (2): an oxidation step of subjecting the dehydrogenation reaction gas obtained in the dehydrogenation step to an oxidation reaction of at least a part of hydrogen in the presence of an oxidation catalyst and in the coexistence of an oxygen-containing gas, and
   Step (3): a dehydrogenation step of subjecting the oxidation reaction gas obtained in the oxidation step to a dehydrogenation reaction of ethylbenzene in the presence of a dehydrogenation catalyst to obtain a dehydrogenation reaction gas comprising styrene, hydrogen, unreacted ethylbenzene, steam, and an alkaline substance derived from the dehydrogenation catalyst.

2. The process for producing styrene according to Claim 1, wherein the blend ratio of the steam in the raw material gas in step (1) is within a range of from 1 to 15 by molar ratio to a raw material hydrocarbon including the ethylbenzene.

3. The process for producing styrene according to Claim 1 or 2, wherein the dehydrogenation catalyst is a potassium-containing iron type catalyst, and the alkaline substance is a potassium compound.

4. The process for producing styrene according to any one of Claims 1 to 3, wherein in the section from the place where the dehydrogenation reaction gas from the outlet of the first dehydrogenation reactor is mixed with the oxygen-containing gas to the inlet of the step (2) oxidation reactor, the temperature of the dehydrogenation reaction gas mixed with the oxygen-containing gas is maintained to be at least the same level as the outlet temperature of step (1), wherein
the temperature difference between the temperature of the dehydrogenation reaction gas mixed with the oxygen-containing gas and the outlet temperature of step (1) is at most 20°C.

5. The process for producing styrene according to any one of Claims 1 to 4, wherein in the section from the place where the dehydrogenation reaction gas from the outlet of the first dehydrogenation reactor is mixed with the oxygen-containing gas to the inlet of the step (2) oxidation reactor, the temperature of the dehydrogenation reaction gas mixed with the oxygen-containing gas is maintained to be at most 560°C.

6. The process for producing styrene according to any one of Claims 1 to 5, wherein in the section from the place where the dehydrogenation reaction gas from the outlet of the first dehydrogenation reactor is mixed with the oxygen-containing gas to the inlet of the step (2) oxidation reactor, the temperature of the dehydrogenation reaction gas mixed with the oxygen-containing gas is maintained to be from 500 to 560°C.

7. The process for producing styrene according to any one of Claims 1 to 6, wherein the temperature-rising width in step (2) is from 10 to 100°C against the temperature of the dehydrogenation reaction gas in the section from the place where the dehydrogenation reaction gas from the outlet of the first dehydrogenation reactor is mixed with the oxygen-containing gas to the inlet of the step (2) oxidation reactor.

8. The process for producing styrene according to any one of Claims 1 to 7, wherein in the section from the place where the dehydrogenation reaction gas from the outlet of the first dehydrogenation reactor is mixed with the oxygen-containing gas to the inlet of the step (2) oxidation reactor, the inner wall surface of the flow channel is made of a material having a nickel content of less than 8 wt%.

**Patentansprüche**

1. Verfahren zur Herstellung von Styrol, welches die folgenden Schritte (1) bis (3) umfasst und welches **dadurch gekennzeichnet ist, dass** zur Zeit des Einspeisens eines Dehydrierungsreaktionsgases, das in Schritt (1) erhalten und mit einem sauerstoffhaltigen Gas vermischt wird, in den Schritt (2) die Umwandlung von Sauerstoff durch Verbrennung auf höchstens 15% reguliert wird in dem Abschnitt von dem Ort, wo das Dehydrierungsreaktionsgas von dem Auslass des ersten Dehydrierungsreaktors mit dem sauerstoffhaltigen Gas vermischt wird, zu dem Einlass des Oxidationsreaktors des Schrittes (2):

Schritt (1): ein Dehydrierungsschritt des Unterziehens eines Ausgangsmaterialgases, das mindestens Ethylbenzol und Dampf umfasst, einer Dehydrierungsreaktion des Ethylbenzols in Gegenwart eines Dehydrierungskatalysators, um ein Dehydrierungsreaktionsgas zu erhalten, umfassend Styrol, Wasserstoff, nicht umgesetztes Ethylbenzol, Dampf und eine alkalische Substanz, abgeleitet von dem Dehydrierungskatalysator,
Schritt (2): ein Oxydationsschritt des Unterziehens des in dem Dehydrierungsschritt erhaltenen Dehydrierungsreaktionsgases einer Oxidationsreaktion mindestens eines Teils des Wasserstoffs in Gegenwart eines Oxidationskatalysators und unter gleichzeitigem Vorhandensein eines sauerstoffhaltigen Gases, und
Schritt (3): ein Dehydrierungsschritt des Unterziehens des in dem Oxidationsschritt erhaltenen Oxidationsreaktionsgases einer Dehydrierungsreaktion von Ethylbenzol in Gegenwart eines Dehydrierungskatalysators, um ein Dehydrierungsreaktionsgas zu erhalten, umfassend Styrol, Wasserstoff, nicht umgesetztes Ethylbenzol, Dampf und eine alkalische Substanz, abgeleitet von dem Dehydrierungskatalysator.

2. Verfahren zur Herstellung von Styrol nach Anspruch 1, wobei das Mischungsverhältnis des Dampfes in dem Ausgangsmaterialgas in Schritt (1) innerhalb eines Bereichs von 1 bis 15 liegt, als Molverhältnis zu einem das Ethylbenzol beinhaltenden Ausgangsmaterial-Kohlenwasserstoff.

3. Verfahren zur Herstellung von Styrol nach Anspruch 1 oder 2, wobei der Dehydrierungskatalysator ein kaliumhaltiger Katalysator vom Eisentyp ist, und die alkalische Substanz eine Kaliumverbindung ist.

4. Verfahren zur Herstellung von Styrol nach mindestens einem der Ansprüche 1 bis 3, wobei in dem Abschnitt von dem Ort, wo das Dehydrierungsreaktionsgas von dem Auslass des ersten Dehydrierungsreaktors mit dem sauerstoffhaltigen Gas vermischt wird, zu dem Einlass des Oxidationsreaktors des Schrittes (2) die Temperatur des Dehydrierungsreaktionsgases, das mit dem sauerstoffhaltigen Gas vermischt wird, so beibehalten wird, dass sie auf mindestens dem gleichen Niveau liegt wie die Auslasstemperatur des Schrittes (1), wobei die Temperaturdifferenz zwischen der Temperatur des Dehydrierungsreaktionsgases, das mit dem sauerstoffhaltigen Gas vermischt wird, und der Auslasstemperatur des Schrittes (1) höchstens 20°C beträgt.

5. Verfahren zur Herstellung von Styrol nach mindestens einem der Ansprüche 1 bis 4, wobei in dem Abschnitt von dem Ort, wo das Dehydrierungsreaktionsgas von dem Auslass des ersten Dehydrierungsreaktors mit dem sauerstoffhaltigen Gas vermischt wird, zu dem Einlass des Oxidationsreaktors des Schrittes (2) die Temperatur des Dehydrierungsreaktionsgases, das mit dem sauerstoffhaltigen Gas vermischt wird, auf höchstens 560°C beibehalten wird.

6. Verfahren zur Herstellung von Styrol nach mindestens einem der Ansprüche 1 bis 5, wobei in dem Abschnitt von dem Ort, wo das Dehydrierungsreaktionsgas von dem Auslass des ersten Dehydrierungsreaktors mit dem sauerstoffhaltigen Gas vermischt wird, zum dem Einlass des Oxidationsreaktors des Schrittes (2) die Temperatur des Dehydrierungsreaktionsgases, das mit dem sauerstoffhaltigen Gas vermischt wird, auf 500 bis 560°C beibehalten wird.

7. Verfahren zur Herstellung von Styrol nach mindestens einem der Ansprüche 1 bis 6, wobei die Temperaturerhöhungsbreite in Schritt (2) 10 bis 100°C beträgt, gegenüber der Temperatur des Dehydrierungsreaktionsgases in dem Abschnitt von dem Ort, wo das Dehydrierungsreaktionsgas von dem Auslass des ersten Dehydrierungsreaktors mit dem sauerstoffhaltigen Gas vermischt wird, zu dem Einlass des Oxidationsreaktors des Schrittes (2).

8. Verfahren zur Herstellung von Styrol nach mindestens einem der Ansprüche 1 bis 7, wobei in dem Abschnitt von dem Ort, wo das Dehydrierungsreaktionsgas von dem Auslass des ersten Dehydrierungsreaktors mit dem sauerstoffhaltigen Gas vermischt wird, zu dem Einlass des Oxidationsreaktors des Schrittes (2) die Oberfläche der inneren Wand des Strömungskanals aus einem Material hergestellt ist, das einen Nickelgehalt von weniger als 8 Gew.-% besitzt.

**Revendications**

1. Procédé de production de styrène, qui comprend les étapes (1) à (3) suivantes, et qui est **caractérisé en ce qu'**au moment de charger un gaz de réaction de déshydrogénation obtenu dans l'étape (1) et mélangé avec un gaz contenant de l'oxygène, dans l'étape (2), la conversion d'oxygène par combustion est régulée pour être d'au plus 15 % dans la section allant de l'endroit où le gaz de réaction de déshydrogénation de l'orifice de sortie du premier réacteur de déshydrogénation est mélangé avec le gaz contenant de l'oxygène à l'orifice d'entrée du réacteur d'oxydation de l'étape (2) :

    étape (1) : une étape de déshydrogénation consistant à soumettre une matière première gazeuse comprenant au moins de l'éthylbenzène et de la vapeur d'eau à une réaction de déshydrogénation de l'éthylbenzène en présence d'un catalyseur de déshydrogénation, pour obtenir un gaz de réaction de déshydrogénation comprenant du styrène, de l'hydrogène, de l'éthylbenzène n'ayant pas réagi, de la vapeur d'eau et une substance alcaline dérivée du catalyseur de déshydrogénation,
    étape (2) : une étape d'oxydation consistant à soumettre le gaz de réaction de déshydrogénation obtenu dans l'étape de déshydrogénation à une réaction d'oxydation d'au moins une partie de l'hydrogène en présence d'un catalyseur d'oxydation et en coexistence d'un gaz contenant de l'oxygène, et
    étape (3) : une étape de déshydrogénation consistant à soumettre le gaz de la réaction d'oxydation obtenu dans l'étape d'oxydation à une réaction de déshydrogénation de l'éthylbenzène en présence d'un catalyseur de déshydrogénation pour obtenir un gaz de réaction de déshydrogénation comprenant du styrène, de l'hydrogène, de l'éthylbenzène n'ayant pas réagi, de la vapeur d'eau et une substance alcaline dérivée du catalyseur de déshydrogénation.

2. Procédé de production de styrène selon la revendication 1, dans lequel le rapport de mélange de la vapeur dans la matière première gazeuse dans l'étape (1) se trouve dans une plage de 1 à 15 en rapport molaire sur une matière première hydrocarbure comprenant l'éthylbenzène.

3. Procédé de production de styrène selon la revendication 1 ou 2, dans lequel le catalyseur de déshydrogénation est un catalyseur de type fer contenant du potassium, et la substance alcaline est un composé de potassium.

4. Procédé de production de styrène selon l'une quelconque des revendications 1 à 3, dans lequel dans la section allant de l'endroit où le gaz de réaction de déshydrogénation de l'orifice de sortie du premier réacteur de déshydrogénation est mélangé avec le gaz contenant de l'oxygène à l'orifice d'entrée du réacteur d'oxydation de l'étape (2), la température du gaz de réaction de déshydrogénation mélangé au gaz contenant de l'oxygène est maintenue pour être au moins au même niveau que la température de sortie de l'étape (1), la différence de température entre la température du gaz de réaction de déshydrogénation mélangé avec le gaz contenant de l'oxygène et la température de sortie de l'étape (1) est d'au plus 20°C.

5. Procédé de production de styrène selon l'une quelconque des revendications 1 à 4, dans lequel dans la section allant de l'endroit où le gaz de réaction de déshydrogénation de l'orifice de sortie du premier réacteur de déshydrogénation est mélangé avec le gaz contenant de l'oxygène à l'orifice d'entrée du réacteur d'oxydation de l'étape (2), la température du gaz de réaction de déshydrogénation mélangé avec le gaz contenant de l'oxygène est maintenue pour être d'au plus 560°C.

6. Procédé de production de styrène selon l'une quelconque des revendications 1 à 5, dans lequel dans la section allant de l'endroit où le gaz de réaction de déshydrogénation de l'orifice de sortie du premier réacteur de déshydrogénation est mélangé avec le gaz contenant de l'oxygène à l'orifice d'entrée du réacteur d'oxydation de l'étape (2), la température du gaz de réaction de déshydrogénation mélangé avec le gaz contenant de l'oxygène est maintenue pour être comprise entre 500 et 560°C.

7. Procédé de production de styrène selon l'une quelconque des revendications 1 à 6, dans lequel l'augmentation de température dans l'étape (2) est de 10 à 100°C par rapport à la température du gaz de réaction de déshydrogénation dans la section allant de l'endroit où le gaz de réaction de déshydrogénation de l'orifice de sortie du premier réacteur de déshydrogénation est mélangé avec le gaz contenant de l'oxygène à l'orifice d'entrée du réacteur d'oxydation de l'étape (2).

8. Procédé de production de styrène selon l'une quelconque des revendications 1 à 7, dans lequel dans la section allant de l'endroit où le gaz de réaction de déshydrogénation de l'orifice de sortie du premier réacteur de déshydrogénation est mélangé avec le gaz contenant de l'oxygène à l'orifice d'entrée du réacteur d'oxydation de l'étape (2), la surface de la paroi intérieure du canal d'écoulement est constituée par un matériau ayant une teneur en nickel inférieure à 8 % en poids.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11080045 A
- JP 2002154991 A
- JP 4020410 A
- JP 60130531 A
- JP 4277030 A
- JP 61225140 A
- JP 11322303 A
- JP 9029095 A